# EUROPÄISCHE PATENTSCHRIFT

(11) **EP 2 004 262 B1**
(45) Veröffentlichungstag und Bekanntmachung des Hinweises auf die Patenterteilung: **14.08.2019**
(21) Anmeldenummer: 07720077.2
(22) Anmeldetag: 13.04.2007
(51) Int. Cl.: A61M 15/00, A61J 1/03

(54) **MEDIKAMENTENMAGAZIN FÜR EINEN INHALATOR, SOWIE MEHRDOSISPULVERINHALATOR**
CONTAINER FOR INHALER, AND MULTIDOSE INHALER
RÉSERVOIR D'INHALATEUR, ET INHALATEUR MULTI-DOSES

(30) Priorität: 13.04.2006 EP 06405163
(43) Veröffentlichungstag der Anmeldung: 24.12.2008
(73) Patentinhaber: BOEHRINGER INGELHEIM INTERNATIONAL GMBH, 55216 Ingelheim am Rhein (DE)
(72) Erfinder: WACHTEL, Herbert, 55218 Ingelheim Am Rhein (DE); GESER, Johannes, 55218 Ingelheim Am Rhein (DE); METZGER, Burkhard P., 55218 Ingelheim Am Rhein (DE); SPALLEK, Michael, 55218 Ingelheim am Rhein (DE); KRUEGER, Michael, 55218 Ingelheim am Rhein (DE); KUNZE, Hubert, 44227 Dortmund (DE); MOSER, Achim, 09130 Chemnitz (DE); MOCK, Elmar, 2013 Colombier (CH); LANCI, Antonino, 3006 Bern (CH); KLOPFENSTEIN, André, 2520 La Neuveville (CH)
(74) Vertreter: Frei Patent Attorneys
(86) Internationale Anmeldenummer: PCT/CH2007/000180
(87) Internationale Veröffentlichungsnummer: WO 2007/118342

(56) Entgegenhaltungen:
- WO-A-01/41846
- WO-A-91/06333
- WO-A-2005/016424
- WO-A1-01/41846
- WO-A2-2005/016424
- DE-A1- 4 400 083
- DE-A1- 4 400 083
- US-A- 5 642 727
- US-A- 5 778 873
- US-A- 6 082 356
- US-A- 6 082 356
- US-A1- 2003 034 271
- US-A1- 2004 206 773
- US-B1- 6 443 307

## Beschreibung

Die Erfindung bezieht sich auf das Gebiet der Medikamentenmagazine, insbesondere auf ein Medikamentenmagazin für eine Inhalationsvorrichtung und einen Mehrdosispulverinhalator gemäss dem Oberbegriff der unabhängigen Patentansprüche.

Bei Medikamentenmagazinen stellt sich das Problem, wie eine Medikamentenkammer, in welcher sich ein Medikament befindet, geöffnet werden kann. Insbesondere für Medikamentenmagazine in Inhalatoren stellt sich die Anforderung, dass bei Medikamenten in Pulverform dieses weder bei der Lagerung noch beim Öffnen zusammengedrückt werden sollte, so dass keine Kompaktierung des Pulvers stattfindet. Zudem sollte ein Inhalator möglichst einfach, günstig und Platz sparend aufgebaut sein.

In DE 44 00 083 wird ein Blister beschrieben, in dem eine einzelne Medikamentenkammer in ihrem Innern einen Dorn oder eine Noppe aufweist. Durch Drücken auf die Hinterseite der Medikamentenkammer wird mit dem Dorn eine Deckfolie durchstochen. Ist der Dorn oder die Noppe zudem in einer Halbschale integriert, so kann eine gewisse Verminderung der Pulverkompaktierung erreicht werden. Beim Ausdrücken wird jedoch die Schale verformt, so dass ein sich neben dem Dorn befindliches Pulver trotzdem zwischen Schale und Folie kompaktiert wird. Zudem wird durch den Dorn, die Noppe oder eine umlaufende Folienwölbung ein Aufreissen der Deckfolie nicht klar definiert. Einerseits ist dadurch die Gefahr vorhanden, dass abgerissene Folienstücke ins Medikament gelangen, andererseits ist durch die nicht genau definierte Öffnung der Medikamentenkammer die Reproduzierbarkeit einer auszugebenden Medikamentenmenge nicht gewährleistet, da Restmengen von Pulver hinter einer unvollständig aufgerissenen Folie verbleiben können.

Mit der in US 6,082,358 offenbarten Vorrichtung soll die Reproduzierbarkeit einzelner Medikamentendosen gewährleistet werden. Dazu wird in eine Medikamentenkammer eines Blisters ein Transferelement eingebracht. Über dieses Transferelement wird ein auf die Rückseite eines Blisters ausgeübte Kraft auf eine ganze Länge einer Blisterdeckfolie übertragen, ohne das im Transferelement befindliche Pulver zu drücken. Dabei reisst die Deckfolie auf der ganzen Länge der Kammer auf. Anschliessend wird das Transferelement mit dem darin enthaltenen Pulver aus dem Blister in einen Entnahmekanal ausgedrückt.

Beim Transferelement handelt es sich typischerweise um ein zylinderförmiges Element. Damit wird die Deckfolie aufgerissen. Auch bei einem mit einer Rippe versehenen Transferelement, wird die Deckfolie aufgerissen und nicht klar definiert aufgeschnitten. Damit keine Folienlappen in den Entnahmekanal reichen, wird das Transferelement entsprechend weit ausgestossen. Die losen Transferelemente werden im Entnahmekanal zurückgehalten. Jeder Medikamentenkammer ist so ein separater Entnahmekanal zugeordnet, in welchem die verbrauchten Transferelemente verbleiben. Gegebenenfalls werden sie in einem Sammelbehälter gesammelt. Ein solcher Aufbau braucht sehr viel Platz. Er ist zudem auch in der Herstellung und im Unterhalt sehr aufwändig, da nicht nur ein Blister sondern eine ganze Einheit ersetzt werden muss.

In US 2004/0206773 wird ebenfalls ein Folienblister offenbart, in welchem ein pulverförmiges Medikament vor Kompaktierung geschützt wird. Dazu weist ein Blister ein schalenförmiges Element auf, in welches das Medikament eingebracht ist. Bei Druck auf die Rückseite des Blisters durchbricht der gegebenenfalls abgeschrägte oder mit Stechpunkten versehene Schalenrand die Deckfolie und die Schale inklusive Medikament wird aus dem Blister ausgestossen. Die Schale gelangt dabei in einen Entnahmekanal. Zudem ist auch eine Entleerung der Schale, je nach Richtung des einfallenden Luftstroms und Position der Schale, erschwert, da der Luftstrom nicht vollständig in die Schale gelangen kann.

WO 2005/016424 zeigt feste Ringmagazine für Pulverinhalatoren. Beidseitig des Magazins angebrachte Folien werden absichtlich angestochen oder durch ein Ausdrücken der im Magazin eingebrachten losen Kolben aufgestossen.

US 6,082,356 betrifft eine Vordosier-Einrichtung für ein pulverförmiges Produkt für eine Abgabevorrichtung für das Produkt und insbesondere für einen Inhalator. Dabei sind in einem Blister Innenstrukturen vorgesehen, welche als Transferelemente für Pulverdosen dienen. Diese Transferelemente müssen innerhalb des Blisters lose sein, damit sie in einen Kanal ausgedrückt werden können und das Pulver transferiert werden kann. Damit die Transferelemente nicht in den Inhalationsbereich gelangen können, ist ein zwischen der Kammer und dem Inhalationsbereich vorgesehener Kanal entsprechend eng dimensioniert.

WO 91/06333 beschreibt einen manuell bedienbaren Dosierer zum Dosieren einer vorbestimmten Pulvermenge, der einen Druckzylinder mit einem Kolben aufweist. Dabei ist ein festes Ringmagazin vorgesehen, das mehrere zylindrische Kammern aufweist, die das Pulver enthalten und an ihren beiden Enden mit Membranen verschlossen sind. Mittels Druckluft wird das Pulver aus der jeweiligen Kammer getrieben.

WO 01/41846 betrifft einen als Inhalationsvorrichtung geeigneten Medikamentenspender mit einer Rückstelleinrichtung zum Rückstellen eines mechanischen Mechanismus' des Spenders nach einer Betätigung desselben.

US 2003/0034271 A1 offenbart eine Vorrichtung zum Schützen und Ausgeben von Medikamenten in fester, Pulver- oder flüssiger Form. Die entsprechenden Blisterpackungen bestehen aus zwei Membranen, wobei eine kuppelförmige Auswölbungen aufweist, die durch die zweite Membran verschlossen sind. In diesen Auswölbungen kann eine innere Kammer vorliegen, die das Medikament enthält und mittels Schneidkanten die zweite Membran öffnen kann.

Es ist deshalb eine Aufgabe der Erfindung, ein Medikamentenmagazin für eine Inhalationsvorrichtung zu schaffen, welches einen einfachen Öffnungsmechanismus in einem Inhalator ermöglicht, und bei welchem ein im Magazin befindliches Pulver beim Öffnen der Kammer im wesentlichen nicht mechanisch belastet wird.

Diese Aufgabe lösen die Medikamentenmagazine mit den Merkmalen des unabhängigen Patentanspruchs. Weitere Ausführungsformen sind in den abhängigen Ansprüchen definiert.

Das erfindungsgemässe Medikamentenmagazin ist zur Verwendung in einem Inhalationsgerät, vorzugsweise in einem Mehrdosispulverinhalator, gedacht und weist mindestens eine Medikamentenkammer auf. Das Magazin wird aus zwei aneinander angebrachten, beispielsweise aneinander gesiegelte Folienbahnen gebildet, wobei die mindestens eine Medikamentenkammer zwischen den Folienbahnen ausgebildet ist. In der Medikamentenkammer kann ein einen Wirkstoff tragendes Pulver eingefüllt sein. Die Medikamentenkammer weist nun eine Innenstruktur auf, welche zum Beispiel ein separates Element, beispielsweise ein durch Spritzgiessen oder Thermoformen hergestelltes Kunststoffteil, ist.

In einer Ausführungsform der Erfindung weist die Innenstruktur an einer Vorderseite ein Mittel zum Öffnen der einen Folienbahn auf. Gleichzeitig umfasst die Medikamentenkammer auch ein Rückhaltemittel, welches die Innenstruktur nach einem Öffnen der Medikamentenkammer am Medikamentenmagazin hält und dafür sorgt, dass die Innenstruktur nicht heraus- oder abfällt und beispielsweise lose in einem Gerät herumliegt. Ein solches Rückhaltemittel wird zum Beispiel durch ein Befestigen der Innenstruktur an einer Folie realisiert, oder auch durch Gestaltung der Innenstruktur, derart, dass diese beim bzw. nach einem Öffnen der Kammer an einer Folie oder Teilen davon ansteht. Durch ein Rückhaltemittel wird nicht nur verhindert, dass die Innenstruktur beispielsweise in einen Inhalationskanal gelangt, auch ein möglicherweise notwendiges Rückführen einer Innenstruktur in einen Bereich zum Weitertransport eines Blisters, kann dadurch sehr einfach gestaltet werden. Beispielsweise wird durch ein einfaches Anheben eines Blisterbandes oder eines Medikamentenmagazins das Band inklusive Innenstruktur aus einem Entnahmekanal entfernt und kann mit einem Band weitertransportiert werden.

Die Innenstruktur weist in ihrem Inneren ein Volumen zur Aufnahme eines pulverförmigen Medikaments auf. Dabei wird durch die Innenstruktur dieses Innenvolumen oder auch die gesamte Medikamentenkammer gegenüber mechanischen Einflüssen von Aussen stabilisiert.

Das Medikamentenmagazin weist den Vorteil auf, dass auch in einem Folienblister ein Pulver vor einem Zusammendrücken und einem damit verbundenen Kompaktieren geschützt ist. Es können für die Herstellung des Folienblisters somit auch sehr dünne und flexible Folien verwendet werden. Dies hat wiederum auch auf den Speicherplatz in einem Inhalationsgerät Einfluss, dadurch dass bei gleichbleibenden Umfang ein Magazin mit einer grösseren Anzahl von Einzeldosen versehen werden kann, oder aber, dass ein Magazin kompakter und vielfältiger in einem Inhalator untergebracht werden kann. Je nach Ausführungsform kann eine Folie jedoch auch sehr stabil und fest sein und beispielsweise vorgängig eingebrachte Vertiefungen beinhalten zur Aufnahme der Innenstrukturen.

Auch können diese Innenstrukturen äussere Anstechmechanismen, wie Inhalationshohlnadeln unterstützen, indem sie als Zentrier- und Anstechhilfe dienen und einen Blister dabei stablisieren. Die Innenstruktur kann dabei mit einem Öffnungsmittel, wie Stechpunkten oder Schneidkanten versehen sein, oder auch aufgrund der Scherfläche zum Öffnen einer Medikamentenkammer beitragen. Innenstrukturen weisen auch im Falle von Medikamentenkammern, welche direkt mit Hilfe einer Innenstruktur geöffnet werden, beispielsweise Schneidkanten und/oder Stechpunkte auf.

Einen Vorteil einer Innenstruktur, welche zum eigentlichen Öffnen einer Medikamentenkammer verwendet wird, ist, dass kein äusserer Öffhungsmechnismus, wie beispielsweise eine Anstechnadel, eine Schneidkante etc., mehr als einmal verwendet wird. Dies trägt wesentlich zu einer hygienischeren Anwendung eines sogenannten Multidose-Gerätes bei. Es ist auch keine Zusatzrolle zum Aufrollen einer abgelösten Folie notwendig oder ein Zusatzreservoir für Abfälle, da ein Medikamentenmagazin vor und nach dem Gebrauch im wesentlichen denselben Umfang bzw. dieselbe Gestalt aufweist, einfach mit geöffneter, entleerter Kammer.

Auch ein Öffnungsmechanismus selber lässt sich einfacher gestalten, indem im wesentlich ein Element vorhanden sein muss, welches Druck auf die Rückseite des Magazins im Bereich einer Medikamentenkammer ausübt. Je nach Gestaltung der Innenstruktur, ist ein Öffnen der Kammer auch durch ein einfaches Rollen über eine Rückseite einer Kammer möglich. Dies vereinfacht den Aufbau eines Inhalationsgerätes auch dadurch, dass ein solcher Druckmechanismus weniger genau als beispielsweise ein Anstechmechanismus sein muss. Diese Eigenschaften führen dazu, dass ein Inhalator entsprechend billiger und kleiner ausgeführt werden kann.

Es ist auch möglich Innenstrukturen zu schaffen, welche voneinander unabhängige Medikamenten-Einfüllöffnungen und Entnahmeöffnung aufweisen. Eine Einfüllöffnung ist bevorzugt gross und gegenüber einer anzubringenden Siegelfolie angeordnet. Ein oder mehrere Entnahmeöffnungen, welche nicht mit der Einfüllöffnung übereinstimmen, welche sich insbesondere auf einer anderen Seite der Innenstruktur befinden, bieten sehr vielfältige Möglichkeiten in Bezug auf beispielsweise den Aufbau eines Inhalationsgerätes, die Dosierung und Beschaffenheit eines Medikamentes etc..

In einer Ausführungsform wird eine Innenstruktur durch Spritzgiessen und beispielsweise einstückig hergestellt. Es ist jedoch auch möglich, die Innenstrukturen aus anderen Materialien zu formen, beispielsweise zu stanzen, insbesondere aus Metallfolien. Die Innenstruktur wird nach der Fertigung an einer Folie eines Folienblisters angebracht, vorzugsweise angesiegelt oder direkt auf die Folie gespritzt. Auch eine einstückige Herstellung einer Folie inklusive Innenstruktur ist möglich.

Für eine platzsparende Anordnung von einzelnen Blistern in einem Medikamentenmagazin, insbesondere in einem streifenförmigen Medikamentenkmagazin, weisen die Innenstrukturen bevorzugt einen rechteckigen Grundriss auf. Je nachdem ob die gegebenen Platzverhältnisse eher in einer Breite oder einer Länge eines Medikamentenmagazins gefordert sind, ist eine Innenstruktur sehr schmal und lang, oder aber eher quadratisch. Werden durch die Herstellung der Blister, beispielsweise der Formung von Vertiefungen in einer Blisterfolie, Anforderungen an das Material gestellt, so werden die Innenstrukturen entsprechend den Gegebenheiten angepasst, beispielsweise als rundere Formen gestaltet.

Die Medikamentenmagazine werden in manchen Ausführungsformen derart in einen Inhalator eingebracht, dass eine Innenstruktur direkt in einen Inhalationskanal ausgedrückt wird. Wird das Ausdrücken nach unten ausgeführt, so fällt ein Pulver aufgrund der Schwerkraft aus der geöffneten Medikamentenkammer. In beiden Fällen wird das Pulver aufgrund eines Luftstromes im Inhalationskanal, z. B. durch eine am Inhalator einatmende Person, mitgenommen, wobei im ersten Fall der Luftstrom das Pulver direkt aus der Innenstruktur auslöst. Ein solcher Luftstrom kann auch im zweiten Fall unterstützend wirken.

Die Innenstruktur ist einerseits so aufgebaut, dass sie eine Kompaktierung des Pulvers bei einem Durchdrücken eines Blisters verhindert. Andererseits ist sie so offen gestaltet, dass sie einen Luftstrom durch die Innenstruktur hindurch, zum Beispiel von mehreren Seiten ermöglicht. Damit ist eine vollständige Entleerung der Innenstruktur und damit eine Reproduzierbarkeit einer anzugebenden Medikamentenmenge gegeben. Mehrere Ein- und Austrittsöffnungen für einen Luftstrom oder Aussparungen an unterschiedlichen Seiten einer Innenstruktur, können zudem die Verwirbelung erhöhen und so eine Deagglomeration von Wirkstoff und Trägermaterial unterstützen.

Die verwendeten Materialien zur Herstellung von Medikamentenmagazinen, insbesondere für Folien und Innenstrukturen, sind bevorzugt pharmazeutisch zugelassene Materialien. Als Folien können Mehrschichtfolien verwendet werden, welche auch zur Herstellung konventioneller Blister geeignet sind. Dies sind in der Regel Mehrschichtfolien aufweisend eine Schicht PE, PP oder PVC und eine Aluminiumschicht. Je nach Anforderung ist die Schicht aufgebaut, beispielsweise stabiler, z.B. als Bodenfolie oder Blister, reissfest, z.B. als peelbare Folie oder stechbzw. durchdrückbar, z.B. als Durchdrückfolie, was zum Beispiel durch den Einbau einer PET Schicht realisiert wird.

In beispielhaften Materialkombinationen weist eine äusserste Schicht (eine innerste bezüglich des Blister) einer ersten Folie, an welcher eine Innenstruktur befestigt wird, die Innenstruktur selber und eine innerste Schicht einer zweiten (Deck-)folie dasselbe Material auf. Dies hat den Vorteil, dass sämtliche Siegelvorgänge, wie Innenstruktur an erster Folie und Deckfolien an Bodenfolie, durch Verschweissen/Verschmelzen derselben Materialien durchführbar sind. Die Innenstrukturen sind dann entsprechend den verwendeten Folien zum Beispiel aus PE, PP oder PET hergestellt.

Es ist auch möglich Siegellack, z.B. Heisssiegellack, zu verwenden. Dadurch sind mehr Materialkombinationen der einzelnen Elemente eines Medikamentenmagazins möglich. Ein Siegellack kann beispielsweise auf Deck- und Innenstruktur oder auch auf eine Bodenfolie aufgebracht werden.

Zum Siegeln wird Wärme an die entsprechenden zu verschweissenden/zu versiegelnden Stellen gebracht. Dies kann durch unterschiedliche Verfahren geschehen, beispielsweise mittels Heissstempel oder auch mittels Induktion, wobei die Aluminiumschicht dabei als Induktionsschicht dienen kann und die Wärme an die umgebende Kunststoffschicht, welche als separate Lackschicht, als integrierte Lack-Folienschicht oder auch als Folienschicht gestaltet sein kann, abgibt. Auch ein Vorheizen der Innenstruktur ist möglich.

In der Regel sind Deckfolien dünner gestaltet als Bodenfolien, so dass ein Wärmeeintrag von aussen, beispielsweise mittels Heissstempel, sehr direkt über eine Deckfolie erfolgen kann.

Die Medikamentenmagazine werden zum Beispiel in einem Mehrdosispulverinhalator eingesetzt. Die Anzahl der im Magazin untergebrachten Einzeldosen liegt vorzugsweise in einem Bereich von 1 bis 100 oder bis 200 Einzeldosen, zum Beispiel in einem Bereich von 1-60, beispielsweise zwischen 7-180 oder 14-150, z.B. 30-120, 45-100, 30, 90, 60, 120. Für Inhalationsgeräte liegt eine Maximalanzahl von Einzeldosen aus Handlichkeits- und Therapie-Gründen beispielsweise bei 60.

Typische Mehrdosispulverinhalatoren sind beispielsweise aus US 5,590,645, US 4,627,432, US 6,655,381 oder WO 2005/002654 bekannt. Darin werden unterschiedlichste Medikamentenmagazine, beispielsweise in der Form von Bändern oder als Ringmagazine, fest oder flexibel, mit integrierten oder separaten Medikamententaschen und entsprechend unterschiedlichen Öffnungsmechanismen, wie Anstechen oder Peelen, in verschiedenen Kombinationen, beschrieben.

Die anspruchsgemässen Blister mit Innenstruktur werden zum Beispiel in solchen Mehrdosispulverinhalatoren eingesetzt. Die in den genannten Schriften verwendeten und beschriebenen Medikamente und Pulver sind Beispiele für die Art, Zusammensetzung und Pulvergrösse von in den anspruchsgemässen Blistern verwendbaren Pulvern und Medikamenten. Die in den Schriften genannten Inhalatoren sind auch bezüglich Grösse, Anwendung und allgemeinem Aufbau Inhalatoren, wie sie sich - bis auf den eigentlichen Öffnungsmechanismus - für die erfindungsgemässen Medikamentenmagazine eignen.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Die unten genannten Verbindungen können allein oder in Kombination zur Anwendung in der anspruchsgemässen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetikum dar, kombiniert mit einem Anticholinergikum, Corticosteroid, PDE4-Inhibitor, EGFR-Hemmer oder LTD4-Antagonisten,
- **W** stellt ein Anticholinergikum, dar, kombiniert mit einem Betamimetikum, Corticosteroid, PDE4-Inhibitor, EGFR-Hemmer oder LTD4-Antagonisten,
- **W** stellt ein Corticosteroid dar, kombiniert mit einem PDE4-Inhibitor, EGFR Hemmer oder LTD4-Antagonisten
- **W** stellt einen PDE4 Inhibitor dar, kombiniert mit einem EGFR-Hemmer oder LTD4-Antagonisten
- **W** stellt einen EGFR-Hemmer dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino] ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino] ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino] ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl} -6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxyethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

### Ebenfalls bevorzugte Anticholinergika sind ausgewählt aus den Salzen der Formel AC-1

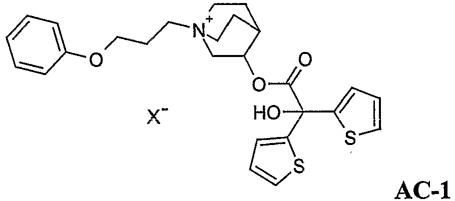

worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 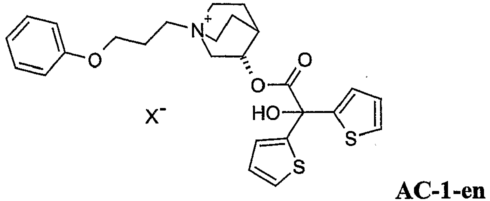 enthalten, worin X ⁻ die vorstehend genannten Bedeutungen aufweisen kann. Weiterhin bevorzugte Anticholinergika sind ausgewählt aus den Salzen der Formel **AC-2** 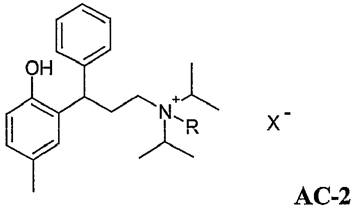 worin R entweder Methyl oder Ethyl bedeuten und worin X ⁻ die vorstehend genannte Bedeutungen aufweisen kann. In einer alternativen Ausführungsform kann die Verbindung der Formel **AC-2** auch in Form der freien Base **AC-2-base** vorliegen.

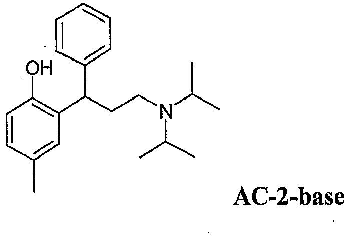

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure(S)-fluoromethylester
- 6,9-Difluor-11-hydroxy-16-methyl-3-oxo-17-propionyloxy-androsta-1,4-dien-17-carbothionsäure(S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R^{∗},10*b*S^{∗})-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yI)-(E)-ethenyl)phenyl)-3-(2-(1-hydroxy-1-methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4- [(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 offenbart.

Weiterhin kann die Verbindung aus der Gruppe der Derivate von Mutterkornalkaloiden, der Triptane, der CGRP-Hemmer, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

Für die Inhalation kommen Arzneimittel, Arzneimittelformulierungen und - mischungen mit den o.g. Wirkstoffen in Betracht, sowie deren Salze, Ester sowie die Kombination dieser Wirkstoffe, Salze und Ester.

Im Folgenden wird der Erfindungsgegenstand anhand von Ausführungsbeispielen, welche in den beiliegenden Zeichnungen dargestellt sind, näher erläutert. Es zeigen jeweils schematisch:
Figur 1a-1e einen Folienblister mit Innenstruktur,
Figur 2a-2c weitere Innenstrukturen,
Figur 3a-3c einen Folienblister mit symmetrischer Innenstruktur,
Figur 4a-c einen Folienblister mit asymmetrischer Innenstruktur,
Figur 5a-5c eine Innenstruktur mit Luftstrom durch das Innere der Innenstruktur,
Figur 6a-6d eine weitere Innenstruktur mit Luftstrom durch die Innenstruktur,
Figur 7a, 7b eine Innenstruktur als Zentrier- und Stechhilfe für Saugnadeln,
Figur 8a, 8b eine weitere Innenstruktur als Zentrier- und Stechhilfe,
Figur 9a, 9b eine Innenstruktur mit zweiseitigen Stechpunkten oder Schneidkanten,
Figur 10a, 10b eine Innenstruktur mit seitlichen Luftzufuhrkanälen,
Figur 11a-c eine Variante der Innenstruktur gemäss den Figuren 1a-e,
Figuren 12a-c, 13a-c zwei Varianten der Innenstrukturen gemäss Figuren 3a-c,
Figuren 14a-c eine Variante der Innenstruktur gemäss Figuren 4a-c,
Figuren 15a-c Blisterformen für Innenstrukturen gemäss den Figuren 11a-c, 12a-c und 13, sowie gemäss der Fig.14a-c.

Grundsätzlich sind in den Figuren gleiche Teile mit gleichen Bezugszeichen versehen. Die beispielhaft beschriebenen Medikamentenmagazine in der Form eines Folienblisters weisen jeweils eine untere und eine obere Folie auf, zwischen welchen eine Innenstruktur eingebracht ist. In den Figuren ist eine erste untere Folie diejenige, in welche die Innenstruktur eingebracht ist und welche dazu in der Regel eine Vertiefung aufweist, bzw. dadurch eine Vertiefung eingeformt wird. Die zweite Folie wird entsprechend als Siegelfolie bezeichnet, da sie in einem Herstellungsprozess zur Versiegelung über einer gefüllten Innenstruktur bzw. Medikamentenkammer angebracht wird. Je nach Material und Herstellungsprozess des Magazins sind die beiden Folien symmetrisch angeordnet. Die Medikamentenkammern sind zum Beispiel Teile eines Medikamentenmagazins mit einer Vielzahl von nebeneinander angeordneten Medikamentenkammern mit Medikamenteneinzeldosen.

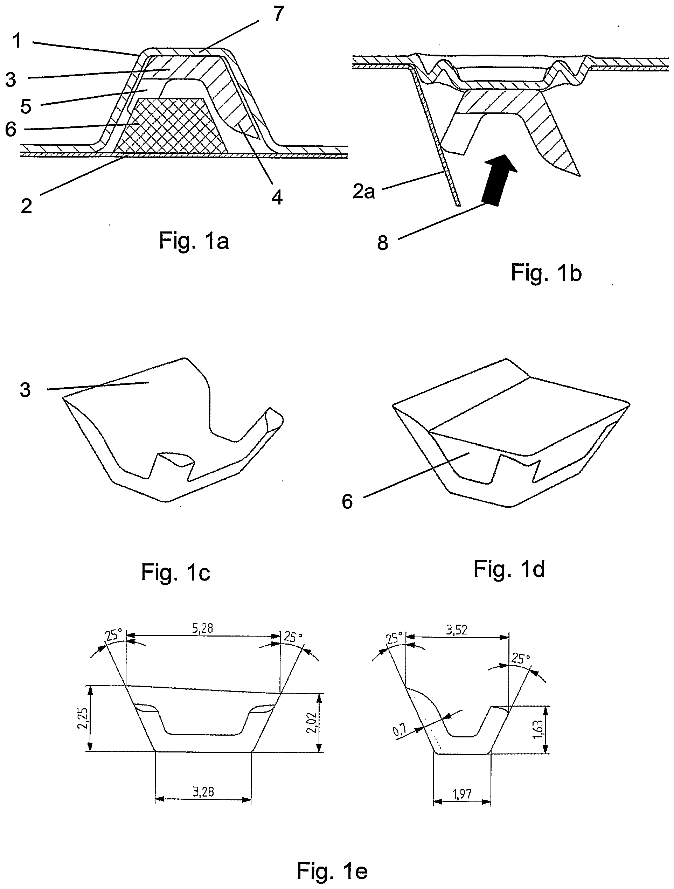

**Figur 1a** zeigt einen Schnitt durch eine nicht anspruchsgemässe Medikamentenkammer in einem Folienblister. Der Blister weist eine erste Folie 1, eine Innenstruktur 3 und eine mit der ersten Folie 1 dicht verschlossene Siegelfolie 2 auf. Die Innenstruktur 3 weist eine Seitenwand mit einer Schneidkante 4 auf, sowie auf der der Schneidkante gegenüberliegenden Seite zwei Beine 5 (**Figur 1c**). Die Beine 5 sind gegenüber der Schneidkante 4 zurückversetzt und unterstützen den Öffnungsvorgang der Medikamentenkammer. Wirkt eine Kraft auf die Rückseite des Blisters, so wird zuerst die Schneidkante 4 gegen die Siegelfolie 2 gedrückt, wobei diese aufgeschnitten wird. Anschliessend drücken die Beine 5 den zuvor von der Schneidkante geöffneten Folienlappen 2a zur Seite, wie in **Figur 1b** gezeigt. Das im Blister vorhandene Pulver 6 kann nun herausfallen und/oder durch einen Luftstrom 8, in der Figur mit einem Pfeil eingezeichnet, dem Blister entnommen werden. Ein Luftstrom kann von oben, zwischen der Seitenwand und den Aufdrückbeinen durch die Innenstruktur hindurch, aber auch seitlich in die Innenstruktur gelangen.

Die Innenstruktur ist an ihrer Rückseite mit der ersten Folie versiegelt. Dies hat den Vorteil, dass die Innenstruktur auch bei vollständig geöffnetem Blister nicht herabfallen und/oder sich als separates Teil verselbständigen kann. Mit dieser Art eines Blisters fällt zudem kein Material an, das separat gesammelt werden muss. Die Innenstruktur kann auch zusätzlich auf den Längsseiten der Innenstruktur versiegelt sein, so dass kein Pulver 6 hinter diese Seitenwandabschnitte gelangen kann.

Die Innenstruktur 3 weist auch eine gewisse Stabilität auf, so dass das in ihm befindliche Pulver 6 vor mechanischen Einflüssen von aussen, insbesondere vor einer Kompaktierung durch Druck, teilweise oder gänzlich geschützt ist.

In den Figuren **1d und 1e** ist eine Schrägansicht und zwei Seitenansichten inklusive einer beispielhaften Vermassung der nicht anspruchsgemässen Innenstruktur für ein beispielhaftes Pulvervolumen von ca. 10mm³ dargestellt. In den Figuren ist zudem zu sehen, dass die Schneidkante leicht geneigt ist, so dass die Siegelfolie zu Beginn der Öffnung von einer Ecke der Schneidkante durchstochen wird und erst anschliessend auf der gesamten Breite der Innenstruktur, entlang der Schneidkante aufgeschnitten wird. Es wäre auch möglich, die Schneidkante durch einen Stechpunkt und eine Querstrebe zu ersetzen. Dabei würde die Folie ebenfalls an einer Ecke aufgestochen, die restliche Breite der Folie würde dabei aber nicht kontrolliert aufgeschnitten, sondern gerissen.

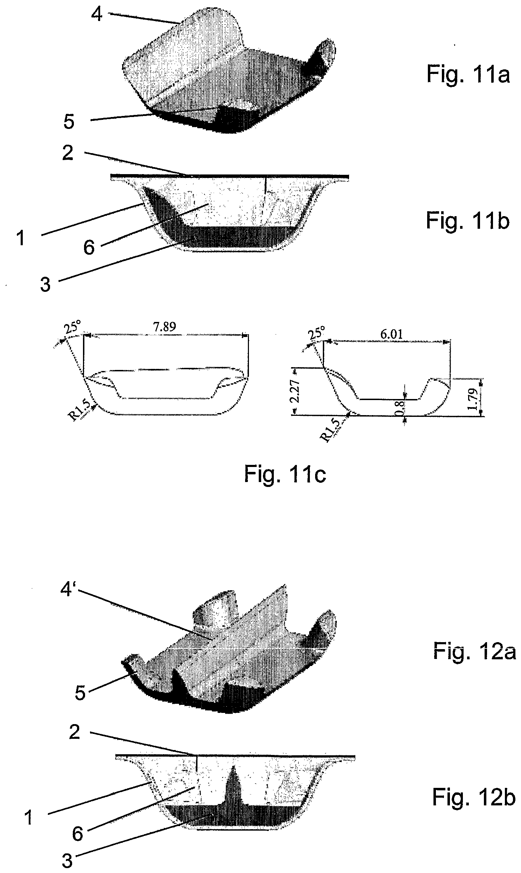

Eine nicht anspruchsgemässen Variante der Innenstruktur gemäss den Figuren 1a-e ist in den **Figuren 11a****-c,** in einer Aufsicht, einer seitlichen Schnittansicht und als vermasster Schnitt, gezeigt. In dieser Variante sind diverse Kanten und Elemente abgerundet bzw. mit grösseren Radien versehen. Dies ermöglicht einen verbesserten Luftstrom durch die Innenstruktur hindurch. Die geänderten Radien an den Beinen verhindern zudem, dass eine Siegelfolie an den Beinen zu reissen beginnt. Die allgemein flachere und breitere Variante erlaubt auch ein höheres Füllvolumen. Das angegebene beispielhafte Volumen der Innenstruktur gemäss Figur 1e beträgt ca. 10 mm³, während die beispielhafte Variante gemäss Figur 11c ein maximales Füllvolumen von ca. 40-45mm³ aufweist.

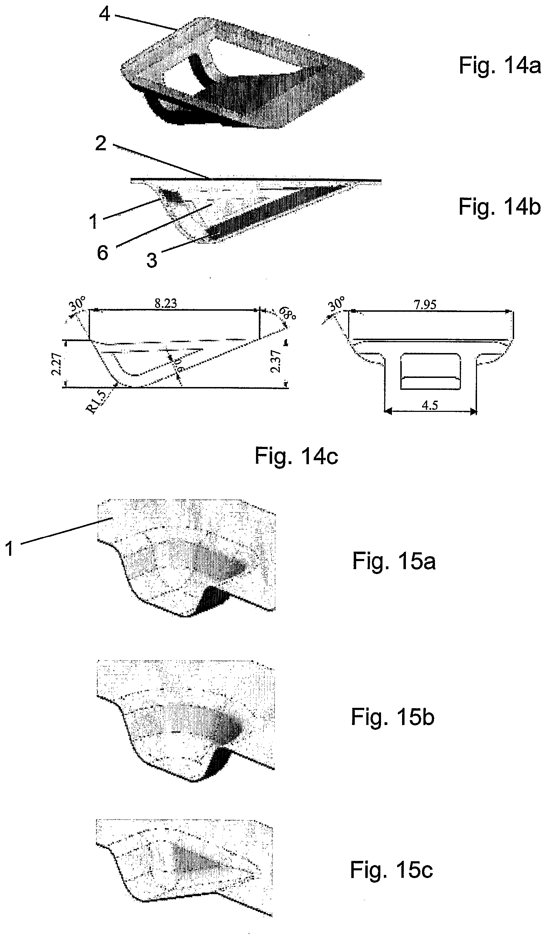

Runde Aussenformen der Innenstruktur sind auch je nach Herstellungsverfahren für ein Magazin bzw. einen Blister möglich. Beispielsweise können einfache Tiefziehverfahren zur Formung von Blisterstrukturen angewendet werden, ohne Streckgrenzen von beispielsweise aluminiumhaltigen Mehrschichtfolien zu überschreiten (siehe dazu auch Figuren 15a-c). Die Blisterformen können damit runder gestaltetet werden, wobei eine korrespondierende Innenstruktur dann vorzugsweise den runden Strukturen des Blisters angepasst ist.

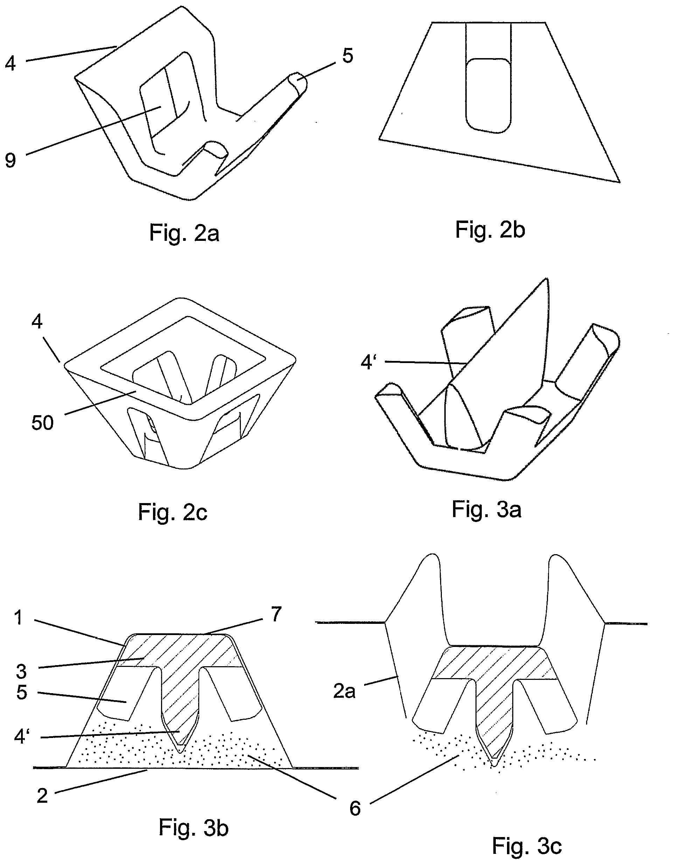

Die Figuren 2a, 2b und 2c zeigen weitere Beispiele von Innenstrukturen mit einer seitlichen Schneidkante und einem im wesentlichen rechteckigen oder quadratischen Grundriss, wobei nur die Innenstrukturen der Figuren 2b und 2c anspruchsgemäss sind. Die Figur 2a weist zudem zwei Aufstossbeine 5 auf. Die Seitenwand, welche die Schneidkante 4 beinhaltet weist eine Öffnung und einen dadurch entstehenden Hohlraum 9 auf. Diese Öffnung bewirkt, dass die Menge von Pulver, welche möglicherweise hinter die Seitenwand gelangt möglichst klein ist: Pulver kann durch die Öffnung wieder in das Innenvolumen der Innenstruktur gelangen und wird nicht zwischen Seitenwand und Folie eingeklemmt.

In der Innenstruktur gemäss den Figuren 2b und 2c sind die Beine als Eckpfeiler ausgestaltet, welche über Querstreben 50 miteinander und mit der Schneidkante verbunden sind. Die Querstreben ermöglichen ein kontrollierteres Schneiden oder Aufdrücken der Siegelfolie, je nachdem, ob die Querstreben zusätzlich als Schneidkanten ausgebildet sind. Die Innenstruktur weist wiederum an unterschiedlichen Ecken unterschiedlich Höhen auf, so dass ein Öffnen der Siegelfolie an einer Ecke beginnt und kontrolliert über die nachfolgenden Kanten bzw. Querstreben verläuft.

In den Ausführungsformen gemäss Fig. 2a-c ist ein Luftstrom längs und quer durch die Innenstruktur möglich. Dadurch ergeben sich auch Verwirbelungsströme, welche eine Deagglomeration eines Medikaments von einem Trägerpulver fördert.

In den Figuren 3a bis 3c ist eine symmetrische Innenstruktur 3 mit mittig angeordneter Schneidkante 4' und entsprechendem Öffnungsvorgang eines Blisters dargestellt, die nicht anspruchsgemäss ist. Die Innenstruktur, hier der Einfachheit halber mit im wesentlichen rechteckigem oder quadratischem Grundriss gezeigt, weist über ihre gesamte Breite eine Schneidkante auf. An allen vier Ecken sind zudem vier gegenüber der Schneidkante leicht zurückversetzte Beine 5 als zusätzliche Öffnungshilfen angebracht.

Das im Blister befindliche Pulver 6 ist durch das durch die Schneidkante und Beine aufgespannte Volumen vor Druck geschützt. Der Blister ist wiederum an seiner Rückseite mit der einen Folie 1 versiegelt, während die Siegelfolie 2 den Blister geschlossen hält. Wird der Blister durch Krafteinwirkung auf seine Hinterseite geöffnet, so schneidet die Schneidkante 4' die Siegelfolie 2 durch, wobei mit Hilfe der Beine 5 zwei Folienlappen 2a entstehen. Diese Folienlappen 2a sind kürzer als bei einer seitlichen Schneidkante und verringern damit das Risiko sich zu lösen, hängenzubleiben oder beim Pulverentnahmevorgang im Weg zu sein. In den Schnittzeichnungen der Figuren 3b und 3c ist wiederum die einseitig erhöhte Schneidkante zu erkennen. Aufgrund der Siegelung 7 an der Rückseite der Innenstruktur bleibt diese an der ersten Folie haften.

Eine symmetrische Form der Innenstruktur weist den Vorteil der einfacheren Herstellung und Handhabung auf. Eine solche Innenstruktur, in welcher die mittige Schneidkante im wesentlichen eine Trennung der Innenstruktur bewirkt, wird beispielsweise so in einen Inhalations- oder Entnahmekanal eingebracht, dass ein Luftstrom parallel zur Schneidkante erfolgt. Die seitlichen Aussparungen zwischen den Beinen unterstützen bzw. ermöglichen jedoch auch einen Luftstrom senkrecht zur Schneidkante.

Ein beispielhaftes Rückhaltemittel ist eine Ansiegelung der Innenstruktur an eine Folie. Die Innenstruktur kann jedoch auch anders an einer Folie befestigt werden, beispielsweise mechanisch, z. B. in der Form einer Niete oder eines Druckknopfes. Dabei weist die Innenstruktur an ihrer hinteren an einer Folie anzubringenden Seite eine Struktur auf, welche mit einer korrespondierenden Form einer Aussenstruktur klemmbar ist. Die eine Folie eines Blisters wird dabei zwischen der Innenstruktur und der Aussenstruktur eingeklemmt. Solche Aussenstrukturen sind beispielsweise integraler Bestandteil eines Medikamentenmagazins und können je nach Ausgestaltung auch noch weitere Funktionen innehaben: Ist ein Medikamentenmagazin bandförmig ausgestaltet, können Aussenstrukturen beispielsweise als Führungen für das Band benutzt werden; Sie können direkt für einen Öffnungs-mechanismus verwendet werden, indem über die Aussenstruktur Druck auf das Magazin und damit auf die Innenstruktur ausgeübt wird; Sie können als Index verwendet werden zum Anzeigen einer Magazinposition etc..

Solche mechanischen, vorzugsweise klemmenden Rückhaltemittel sind dann vorteilhaft, wenn eine Siegelung nicht möglich oder erwünscht ist oder wenn weitere Funktionen, wie beispielsweise oben genannt, in ein Medikamentenmagazin integriert werden sollen.

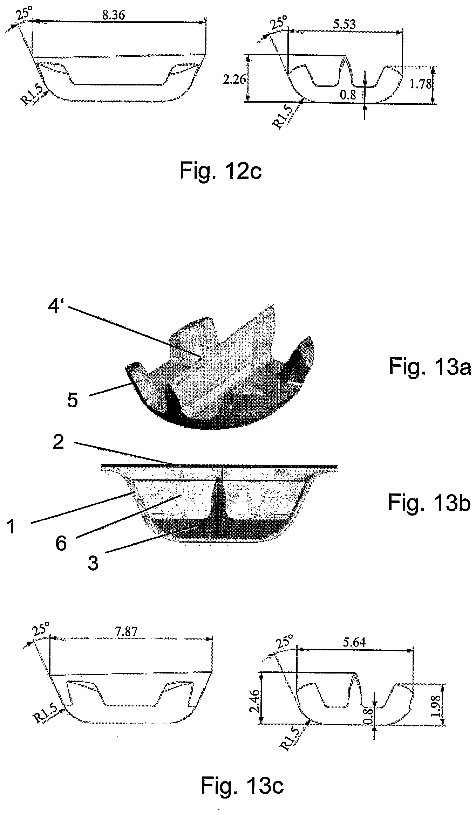

Zwei Varianten der Innenstruktur gemäss den Figuren 3a-c sind in den **Figuren 12a****-c und 13 a-c,** jeweils in einer Aufsicht, einer seitlichen Schnittansicht und als vermasste Schnitte gezeigt. Diese Varianten zeichnen sich wiederum durch ihre abgerundeten Kanten und Beinradien aus. Durch die im wesentlichen quadratische oder rechteckige Grundfläche der Innenstruktur ist eine Ausrichtung derselben beim Ein- oder Aufbringen der Struktur auf oder in eine erste Folie gegeben. In den Figuren 13a-c ist die Grundfläche der Innenstruktur fast vollständig abgerundet. Eine vollständig runde Grund- oder Stirnfläche ermöglicht beispielsweise ein einfacheres Einbringen einer Innenstruktur in eine vorgeformte erste Folie, da keine Ausrichtung notwendig, eine solche aber auch schwerer möglich ist.

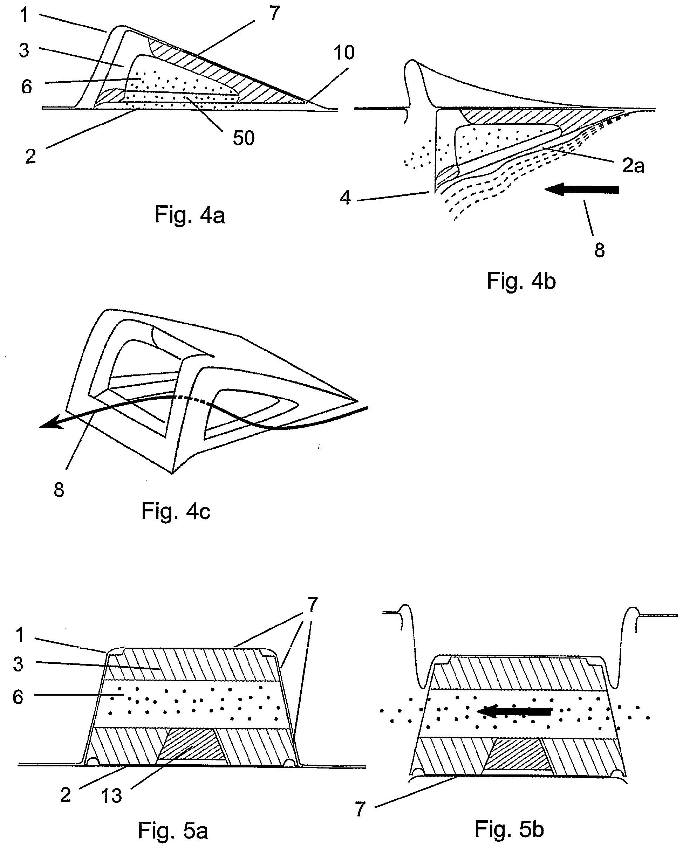

In den **Figuren 4a bis 4c** ist ein Blister mit Innenstruktur gezeigt, welche einerseits asymmetrisch ist und nebst einer Siegelung 7 an ihrer Rückseite auch eine andere Art eines Rückhaltemechanismus erlaubt. Die Innenstruktur in dieser Ausführungsform hat im wesentlichen den seitlichen Querschnitt eines rechtwinkligen Dreiecks, welcher Querschnitt das Öffnen des Blisters durch ein Drücken auf die Hinterseite des Blisters ermöglicht. Aufgrund der rampenartigen Dreieckform der Rückseite der Innenstruktur ist ein Drücken parallel zu und entlang des Folienblisters (welcher in der Zeichenebene von rechts nach links verläuft) möglich. Dies kann sehr einfach durch ein Drüberrollen, beispielsweise einer mit Druck beaufschlagten Rolle, über die Hinterseite des Blisters ausgeführt werden. Es ist auch möglich, dass ein Blisterband beim Weitertransportieren an einem z. B. rampenförmigen Element 'ausgedrückt' wird (dabei würde sich das Band entsprechend von links nach rechts bewegen). Damit kann eine Vorrichtung kompakter gestaltet werden, da beispielsweise kein Hub eines Krafteinwirkungselements nötig ist.

Durch die Kraft, die auf die schräge Rückseite des Blisters einwirkt, schneidet eine auf der Vorderseite der Innenstruktur 3 befindliche Schneidkante 4 in die Siegelfolie 2 und gräbt sich, ähnlich einer Baggerschaufel, weiter durch diese. Querstreben 50, die von der Schneidkante im wesentlichen parallel zur Siegelfolie 2 verlaufen und welche vorzugsweise auch als Schneidkanten ausgestaltet sind, schneiden die Siegelfolie auf ihrer gesamten Länge und führen zu einem länglichen Folienlappen 2a. Ist die Innenstruktur ganz aus dem Blister bzw. der Medikamentenkammer ausgedrückt, fällt das in der Innenstruktur befindliche Pulver zumindest teilweise aufgrund der Schwerkraft in einen Inhalationsraum (nicht dargestellt). Es ist auch möglich, dass das Pulver gegebenenfalls zusätzlich durch einen im Inhalationsraum wirkenden Luftstrom 8, welcher in den Figuren 4b und 4c durch einen Pfeil eingezeichnet ist, aus der Innenstruktur gelöst wird. Der Folienlappen 2a kann dabei eine unterstützende Funktion beim Auslösen des Pulvers haben, indem der Lappen 2a durch den Luftstrom 8 gegen die Unterseite der Innenstruktur vibriert, und dabei ein Ausklopfen des Pulvers bewirkt. Eine Bewegung des Folienlappens 2a ist in der Figur gestrichelt eingezeichnet. Seitliche und eine vordere Öffnung in der Innenstruktur ermöglichen einen Luftstrom 8 durch die Innenstruktur, wie in Figur 4c eingezeichnet. Dieser sorgt für ein vollständige Auslösen der Pulvers und eine Verwirbelung für eine bessere Loslösung eines Wirkstoffes von einem Trägermaterial (Dispersion).

Um zu Verhindern, dass die Innenstruktur aus dem Blister fällt, kann anstelle oder zusätzlich zur Siegelung 7 an der Rückseite der Innenstruktur an der ersten Folie auch beispielsweise eine Art Scharnier 10 durch Folie(n) und Innenstruktur gebildet sein. Dabei wird die Innenstruktur 3 mit einer Drehbewegung aus dem Blister gedrückt. Zur Bildung eines solchen Scharniers 10 könnte beispielsweise diejenige Kante der Innenstruktur, an welcher Hypothenuse und Ankathete des Dreiecks zusammentreffen, derart verlängert sein, dass die Kante zwischen den beiden Folien festgemacht, z. B. eingeklebt, eingeschweisst oder eingesiegelt ist.

Die in den Figuren 4a-4c gezeigte Innenstruktur wäre auch geeignet, um aus einer Metallfolie geformt, z. B. gestanzt und gefaltet, zu werden.

Nicht nur das Ausdrücken der Innenstruktur kann in dieser Ausführungsform sehr kompakt ausgeführt sein. Auch das Rückführen der Innenstruktur zurück auf eine Ebene in unausgedrücktem Zustand des Blisters, beispielsweise zwecks Weitertransport eines Blisterbandes, kann durch ein Anpressen der Innenstruktur an ein z.B. rampenartiges Element erfolgen. Dadurch können während einem Weitertransport des Blisterbandes mehrere Aktionen gleichzeitig erfolgen: der Transport und das Rückführen der Innenstruktur in seine ursprüngliche Position, gegebenenfalls auch das Ausdrücken eines neuen Blisters.

In dieser Figur entspricht eine typische Bewegungsrichtung eines Blisterbandes auch einer, gegebenenfalls entgegengesetzten, Entnahmerichtung. In beispielhaften Ausführungsformen eines Inhalators, wird eine Entnahme aus Platzgründen im wesentlichen senkrecht zu einer Bewegungsrichtung eines Medikamentenmagazins angeordnet sein.

Eine Variante der Innenstruktur gemäss den Figuren 4a-c ist in den **Figuren 14a****-c,** jeweils in einer Aufsicht, einer seitlichen Schnittansicht und als vermasster Schnitt, gezeigt. In dieser Ausführungsform ist insbesondere die Rückseite und die Ecken und Kanten der Grundfläche der Innenstruktur abgerundet. Zudem weist die Grundfläche eine grössere Breite auf als die Rückseite. Die Innenstruktur bildet hinter einer geöffneten Folie einen Rahmen für ein darin befindliches Pulver oder allgemein druckempfindliches Medikament. Auch hier ist die Schneidkante gegenüber der zu schneidenden Folie leicht geneigt, so dass die Folie zuerst punktförmig angestochen und dann entlang der Schneidkante kontrolliert aufgeschnitten wird.

In den **Figuren 5a** und **5b** ist ein Schnitt durch einen Blister 1 mit einer schachtelförmigen Innenstruktur 3 gezeigt, die nur der Erläuterung dient. Die Schachtel weist in der Mitte einer Vorderseite eine Einfüllbohrung 13 zum Einfüllen eines Pulvers 6 auf, welche nach dem Füllen mit einem Stopfen verschlossen wird. Die Innenstruktur ist derart zwischen zwei Folien eingebracht, dass eine obere Siegelfolie 2 die Einfüllbohrung verschliesst, so dass gegebenenfalls auf einen Stopfen verzichtet werden kann. Das eingefüllte Pulver 6 befindet sich nun vorzugsweise vollständig in einem Kanal im Inneren der Innenstruktur. Dieser Kanal verläuft längs durch die Innenstruktur und ist mit der Einfüllbohrung 13 verbunden.

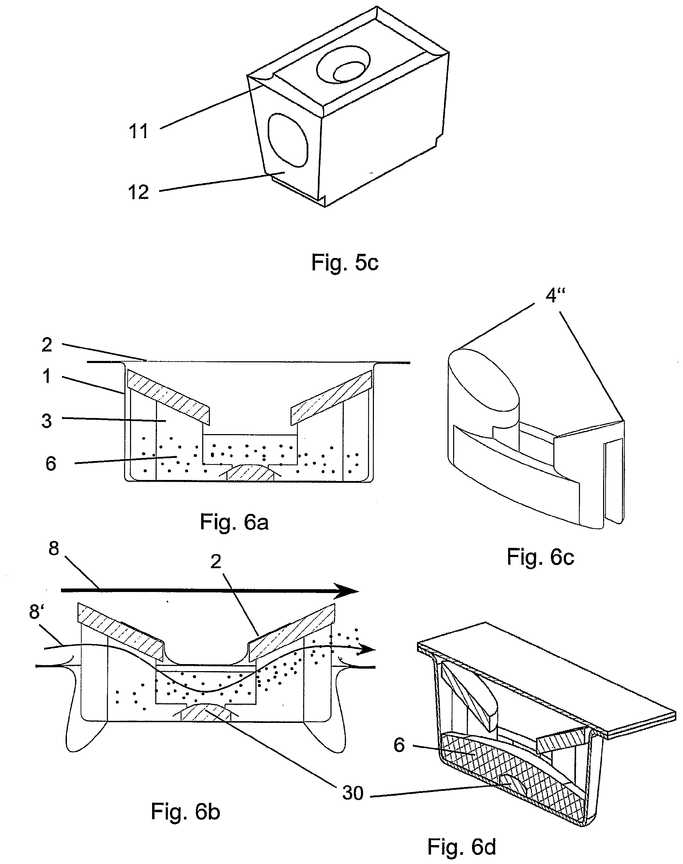

Schneidkanten 11, welche auf der Vorderseite und rings um die Innenstruktur verlaufen, siehe **Figur 5c**, schneiden ein Loch in die Siegelfolie 2, welche der Fläche der Innenstruktur entspricht. Aufgrund einer Siegelung 7 auf der Vorderseite der Innenstruktur bleibt das ausgeschnittene Folienstück an der Innenstruktur haften. Der geöffnete Blister wird nun beispielsweise derart positioniert, dass die Innenstruktur sich in einem Inhalationskanal befindet. Ein Luftstrom im Inhalationskanal, mit einem Pfeil eingezeichnet, führt durch den Kanal in der Innenstruktur und nimmt dabei das darin befindliche Pulver 6 mit. Durch die Siegelfolie 2 auf der Innenstruktur 3 und/oder durch den Stopfen wird verhindert, dass Pulver an einer anderen Stelle als durch die Öffnungen des Kanals, den Austrittsöffnungen, austreten kann.

Eine weitere Siegelung 7 ist wiederum zwischen erster Folie und Rückseite der Innenstruktur vorhanden, um ein Abfallen der Innenstruktur von der Folien zu verhindern. Eine Siegelung 7 der ersten Folie 1 kann aber auch auf denjenigen Seiten der Innenstruktur angebracht sein, welche die Austrittöffnungen aufweisen. Dadurch gerät kein Pulver ausserhalb der Innenstruktur in die Medikamentenkammer. Die seitlichen Siegelungen werden beim Öffnungsvorgang des Blisters von der Innenstruktur abgelöst, so dass die Austrittsöffnungen freigegeben werden.

In der beschriebenen Ausführungsform der Innenstruktur ist ein Pulver vollständig vor einem Zusammendrücken, bei der Lagerung, aber auch bei einem Öffnen des Blisters geschützt. Zudem erlaubt eine von einer oder mehreren Austrittsöffnungen unabhängige Einfüllöffnung mehr Spielraum in Bezug auf eine Entnahme bzw. eine Dosierung eines Medikaments. Diese werden wesentlich durch die Austrittsöffnungen, allgemein durch den Entnahmewiderstand aus der Innenstruktur, bestimmt. Eine Einfüllöffnung ist jedoch vorzugsweise möglichst gross, so dass ein Einfüllen schnell und einfach vorgenommen werden kann. Zudem sollte eine Einfüllöffnung auf einer oberen Seite eines Blisters sein, während Entnahmeöffnungen, wie im vorliegenden Fall, je nach Inhalatoranordnung seitlich angeordnet sein können.

Eine nicht beanspruchte Ausführungsform, welche eine von Austrittsöffnungen unabhängige Einfüllöffnung in einer Innenstruktur aufweist, ist in den **Figuren 6a bis 6d** gezeigt. Ein Pulver 6 wird wiederum durch eine der Siegelfolie 2 gegenüberliegende Einfüllöffnung eingefüllt. Rückwärtige und seitliche Öffnungen sind mit einer ersten Folie 1 verschlossen, wobei die seitlichen Öffnungen beim Öffnungsvorgang von der Folie 1 abgelöst werden und einen Durchgang durch die Innenstruktur zum Durchströmen von Luft freigeben. In der Figur 6b ist ein über die Struktur führender Luftstrom 8, sowie ein durch die Innenstruktur führender abgelenkter Luftstrom 8' eingezeichnet. In der Figur 6c sind zwei bogenförmige Schneidkanten 4" zu sehen, welche zwei voneinander beabstandete Öffnungen in die Siegelfolie 2 schneiden. Bei einem Herausdrücken der Innenstruktur aus dem Blister wird die Siegelfolie in einem Zwischenbereich zwischen den Schneidkanten an die Innenstruktur gedrückt (siehe Fig. 6b), so dass damit einerseits die Einfüllöffnung verschlossen wird und andererseits die Innenstruktur im Blister gehalten wird, ohne dass eine Rückseite versiegelt sein muss. Die Innenstruktur weist im Pulverbereich ein Verwirbelungselement 30 auf, welches für zusätzliche Turbulenzen bei der Entnahme des Pulvers sorgt und dadurch eine Deagglomeration des Pulvers unterstützt.

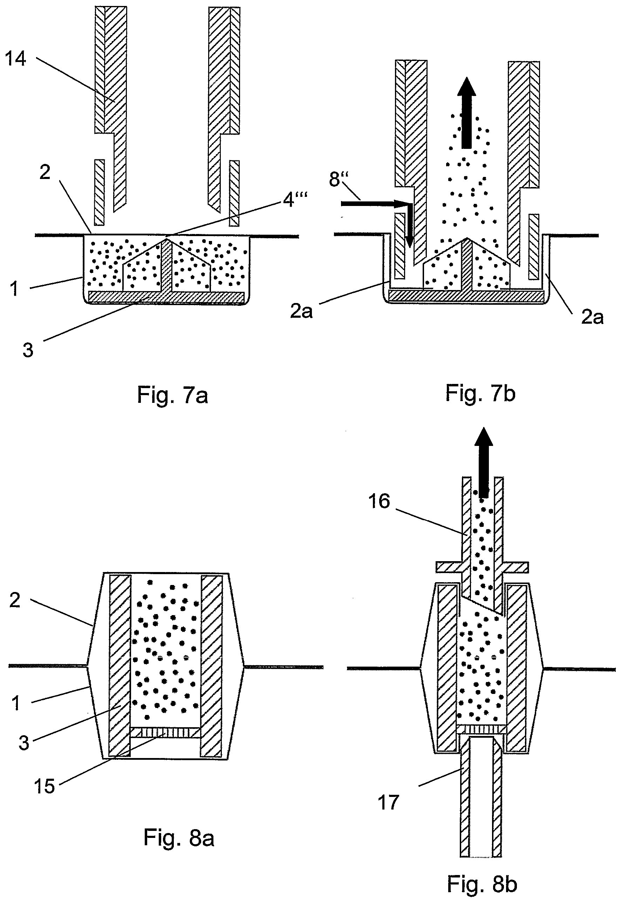

In den **Figuren 7a, 7b**, **8a und 8b** sind nur zur Erläuterung dienende Innenstrukturen dargestellt, welche nebst einer Stabilisierungsfunktion, auch Stech- und Zentrierhilfen für von aussen in den Blister 1 einzuführende Saugnadeln, Kanülen, Luftzufuhrnadeln etc. sind.

In der Figur 7a und 7b ist die Innenstruktur 3 im wesentlichen symmetrisch und weist einen zentral angebrachten Stechpunkt oder eine Schneidkante 4"' auf. Die Innenstruktur 3 ist in Kombination mit einer Doppelkanüle 14 gezeigt. Die doppelwandige Kanüle ist so ausgeführt, dass durch ihr Inneres gesaugt werden kann (dicker Pfeil), während durch den Aussenteil die Luftzufuhr 8" garantiert wird. Die Innenstruktur besteht aus einer Platte, welche in etwa denselben Durchmesser aufweist wie die Kanüle 14. Im Zentrum der Platte sind zur Mitte hin konisch zulaufende Wände aufgebracht, welche als Führung bzw. Anstosspunkte für die einzuführende Kanüle dienen. Dazu weisen die Wände einen leicht grösseren Durchmesser auf als der Innendurchmesser der Kanüle.

An die erste Folie 1, welche vorgeformt sein kann, wird die Innenstruktur angespritzt, angesiegelt oder auf andere Art eingebracht. Vorzugsweise wird beim Anbringen der Innenstruktur eine entsprechende Vertiefung in die Folie 1 geformt. Anschliessend wird die noch offene Medikamentenkammer gefüllt und mit einer Siegelfolie 2 versiegelt. Beim Anstechen mit der Kanüle 14 wird die Siegelfolie aufgrund der auf sie einwirkenden Kraft in der Mitte des Blisters durch die Innenstruktur angestochen und aufgerissen bzw. -geschnitten. Die freien seitlichen Folienlappen werden durch die Doppelkanüle zur Seite, in die Medikamentenkammer und auf die Platte der Innenstruktur hinuntergedrückt. Ein Luftstrom 8' kann nun von aussen durch die Kanüle, durch die Innenstruktur bzw. an der Innenstruktur vorbei, im Inneren entlang der Kanüle in Saugrichtung nachgeführt werden, wobei dadurch eine im wesentlichen vollständige Entnahme des Pulver möglich ist.
Die Innenstruktur 3 in den Figuren 8a und 8b weist selber keine Stechpunkte oder Schneidkanten auf. Die Innenstruktur besteht im wesentlichen aus einem Rohr oder auch zwei gleich grossen parallelen Wänden, welche in einem unteren Teil über einen Filter 15 verbunden sind. Die Innenstruktur ist vorzugsweise ein Kunststoffteil und der Filter 15 ein integraler Bestandteil davon, welcher im wesentlichen eine mit Filteröffnungen versehene dünne Kunststoffwand ist. Der Filter verhindert das Austreten des im Blister befindlichen Pulvers in ein Gerät bzw. das Eintreten von unerwünschten Kleinteilen in die Kammer durch eine Luftzufuhrnadel 17 (siehe unten).

Die Innenstruktur ist zur Verwendung mit einer Saug- 16 und einer Luftzufuhrnadel 17 ausgelegt. Dazu werden die beiden Folien von oben und unten angestochen, wobei die Saug- und Luftzufuhrnadel eine entsprechende Schneidkante bzw. Stechpunkte aufweist. Der Durchmesser des Rohres ist dabei so bemessen bzw. die beiden Wände der Innenstruktur sind soweit voneinander beabstandet, dass die Nadeln 16, 17 dazwischen eingeführt werden können. Beim Aufschneiden der Folien 1, 2 werden die dabei entstehenden Folienlappen zur Seite zwischen Nadel und Folie bzw. Wand gedrückt, wo sie Nadeln und Medikamentenkammer gegeneinander abdichten.

Während die Luftzufuhrnadel 17 am Filter ansteht, weist die Saugnadel 16 vorzugsweise einen Haltemechanismus auf, beispielsweise einen ringförmigen Rand um die Saugnadel 16, welche an den Wänden der Innenstruktur ansteht und ein definiertes Eindringen der Saugnadel in die Innenstruktur ermöglicht.

Die Innenstruktur könnte anstelle der zwei Wände oder des Rohres auch ein Rechteckzylinder sein, so dass ein in seinem Inneren befindliches Medikament im wesentlichen vollständig eingeschlossen ist. Runde Formen der Innenstruktur werden insofern bevorzugt, da ihre Herstellung, Zentrierung einer Nadel oder ihre Ausrichtung in Bezug auf die Folie vereinfacht sind. Obere und untere Folie können auf der Innenstruktur angesiegelt sein.

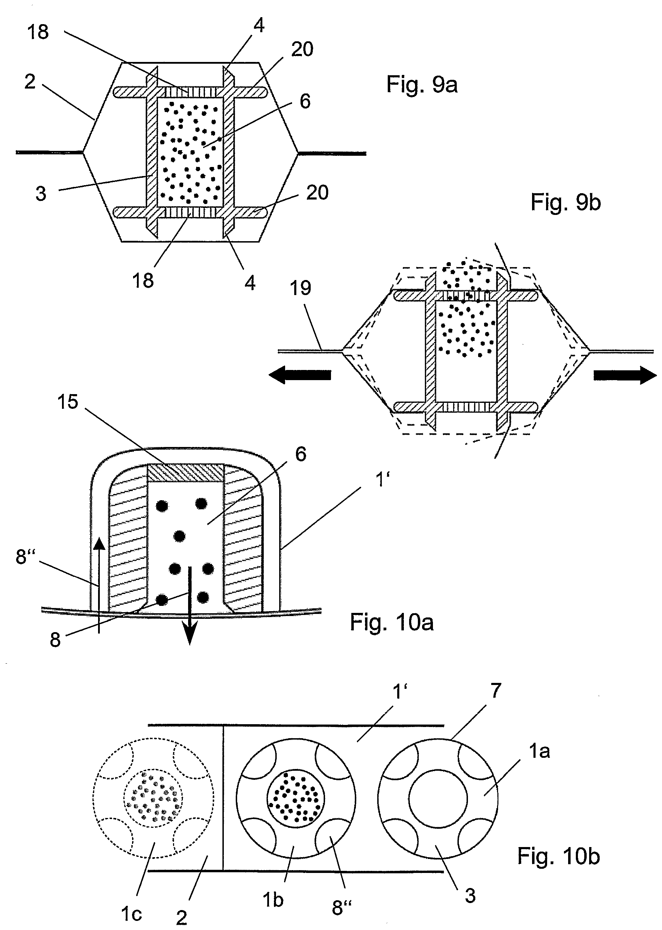

In den nur der Erläuterung dienenden **Figuren 9a und 9b** ist eine runde oder eckige käfigartige Innenstruktur 3 gezeigt, welche nicht an einer Folie angesiegelt wird. Als Rückhaltemechanismus, damit die Innenstruktur auch nach dem Öffnen des Blisters nicht heraus- oder abfällt, weist diese ausserhalb der eigentlichen, das Pulvervolumen umfassenden, Innenstruktur, Vorsprünge 20 auf. Das Innenvolumen wird wiederum durch einen runden Zylinder, zwei parallele Wände oder einen rechteckigen Zylinder aufgespannt. Die Wände bzw. Zylinder weisen an ihren Enden Stechpunkte oder Schneidkanten 4 auf, welche die obere und untere Folie 2 durchtrennen. Zwischen den Wänden bzw. im Inneren der Zylinder sind, vorzugsweise integral, Siebe 18 geformt, welche gröbere Partikel im Innenvolumen zurückhalten.

Die Vorsprünge sind ausserhalb der oberen und unteren Schneidkanten 4 angebracht und als Verlängerungen der Siebe 18 gestaltet.

Figur 9a zeigt einen Blister in ungeöffnetem, Figur 9b in geöffnetem Zustand. Ein Öffnen dieses Blister kann durch ein seitliches Ziehen in Pfeilrichtung an den Laschen 19 geschehen. Verschiedene Stadien des Öffnungsvorganges sind in Figur 9b durch gestrichelte Folienpositionen angedeutet.

In den nur der Erläuterung dienenden **Figuren 10a und 10b** ist eine Innenstruktur 3 mit mehreren Luftzufuhrkanälen gezeigt. Ein Pulver 6 befindet sich im zylindrischen Innenvolumen der im wesentlichen zylinderförmigen Innenstruktur. Der Innenzylinder ist auf einer Seite mit einem Filter 15 versehen, so dass keine Fremdkörper über die Luftzufuhr 8" in ein Pulver und damit in einen Inhalationskanal gelangen. In Figur 10b ist eine Aufsicht auf ein Blisterband 1' mit drei Medikamentenkammern in verschiedenen Gebrauchsstadien dargestellt. Eine Medikamentenkammer oder Blister 1a ist leer, wobei sich die Innenstruktur weiterhin im Blisterband 1' befindet, beispielsweise durch eine Siegelung 7 von Seitenwänden der Innenstruktur mit dem Blisterband 1'. Ein zweiter Blister 1b ist geöffnet und zur Entnahme des Pulvers bereit. Ein dritter Blister 1c ist noch mit einer Siegelfolie 2 verschlossen.

Die Kanäle für die Luftzufuhr können auch filterseitig in die Innenstruktur hineinversetzt sein, derart, dass auch ein Andrücken einer hinteren Folie die Kanäle nicht beinträchtigt oder die Luftzufuhr unterbinden kann.

Das Öffnen der Medikamentenkammer kann durch abziehen, abschaben oder auch aufstechen der Folie im Bereich der Öffnungen geschehen.

Eine in dieser Figur beschriebene Innenstruktur stabilisiert eine Medikamentenkammer und schützt das in ihr befindliche Pulver vor einem Zusammendrücken. Die Entnahme des Pulvers erfolgt jedoch ohne ein Ausdrücken der Innenstruktur aus der Folie. Das Blisterband 1' kann deshalb auch eine sehr dünne, weiche Folie sein, da die Medikamentenkammer durch die Innenstruktur stabilisiert, insbesondere auch die Luftkanäle durch die Innenstruktur freigehalten werden. Umgekehrt kann auch eine sehr stabile und gegebenenfalls für ein Ausdrücken ungeeignete Folie verwendet werden. Dies würde beispielsweise ein Formen dieser Folie ermöglichen, welche Form aufgrund ihrer Stabilität ein Offenhalten der Luftzufuhrkanäle unterstützen kann.

In den **Figuren 15a****-c** sind beispielhafte Blisterformen gezeigt, in welche unterschiedliche Innenstrukturen eingebracht werden können. Die Blisterform gemäss Figur 15a eignet sich beispielsweise für die Innenstruktur gemäss den Figuren 11a-c und 12a-c, die Blisterform gemäss Figur 15b für die Innenstruktur gemäss den Figuren 13a-c und die Blisterform gemäss Figur 15c für die Innenstruktur gemäss Figur 14 a-c.

Die Blisterformen können durch unterschiedliche Verfahren hergestellt werden. Besonders geeignet sind Tiefzieh- und Thermoformverfahren.

Je nach verwendeter Folie sind beim Herstellungsverfahren Streckgrenzen des Materials zu beachten. Niedrigere Streckgrenzen können durch rundere Formen der Innenstrukturen ausgeglichen werden. In den angegebenen Beispielen ist dies mit den Innenstrukturen gemäss den Figuren 11-13 gegeben. Kritische Punkte sind (beispielhafte Werte der Innenstrukturen der Fig. 11-13 sind in Klammern angegeben): möglichst grosse Radien (1.5mm), deutlicher Anzugswinkel an der Wand (25°), Verhältnis Breite zu Tiefe (>2.7).

Es wurden Siegelversuche für Innenstrukturen an Folien durchgeführt. Dabei zeigte sich, dass mit kommerziell erhältlichen Folien für pharmazeutische Produkte sehr gute Ergebnisse erzielt werden. Für die Versuche wurden insbesondere unterschiedliche Mehrschichtfolien verwendet, welche auch in der Herstellung konventioneller Blister Anwendung finden. Die Deckfolien (Durchdrück- und Abziehfolien) wiesen unterschiedliche Aluminiumdicken von 20-40*µ*m auf Zudem wurden Folien mit PVC oder PP Schichten in Kombination mit Heisssiegellack und Folien mit Polymerschichten (LDPExtr) verwendet. Die verwendeten Bodenfolien, welche prinzipiell stabiler gestaltet sind als Deckfolien, wiesen eine Aluminiumschicht im Bereich von ca. 45-47*µ*m und eine Polymerschicht (oPA) von ca. 25 *µ*m auf. Die Bodenfolien unterschieden sich im wesentlichen durch eine zur Siegelung verwendete Schicht aus PVC, PP oder PE. Die Dicken der PVC und PP Schichten lagen in einem Bereich von 60*µ*m, während PE Schichten ca. 40*µ*m Dicke aufwiesen.

## Patentansprüche

1. Medikamentenmagazin mit mindestens einer Medikamentenkammer zur Verwendung in einer Inhalationsvorrichtung, wobei das Magazin aus zwei aneinander angebrachten Folienbahnen (1; 2) gebildet ist, und zwischen den Folienbahnen (1; 2) die mindestens eine Medikamentenkammer ausgebildet ist, wobei die Medikamentenkammer eine Innenstruktur (3) aufweist, welche Innenstruktur (3) in ihrem Inneren ein Volumen zur Aufnahme eines pulverförmigen Medikaments (6) aufweist und dieses Innenvolumen gegenüber mechanischen Einflüssen von Aussen stabilisiert, und wobei die Innenstruktur (3) an einer Vorderseite ein Mittel zum Öffnen (4; 11) einer der zwei Folienbahnen (1; 2) aufweist, wobei das Mittel zum Öffnen mindestens einen vorstehenden Stechpunkt oder eine Schneidkante (4,4', 4", 4"') und vorstehende, in Bezug auf den Stechpunkt oder die Schneidkante zurückversetzte Öffnungshilfen umfasst, und wobei im Medikamentenmagazin ein Rückhaltemittel (7) vorhanden ist, welches die Innenstruktur (3) nach einem Öffnen der Medikamentenkammer am Medikamentenmagazin hält **dadurch gekennzeichnet, dass** von einer die Schneidkante (4) oder den Stechpunkt aufweisenden Seitenwand unter einem Neigungswinkel zwei parallele Querstreben (50) zu einer der genannten Seitenwand gegenüberliegenden Seite der Innenstruktur (3) verlaufen.

2. Medikamentenmagazin nach Anspruch 1, wobei die Innenstruktur (3) mindestens eine seitliche Öffnung oder Aussparung aufweist, derart, dass ein Luftstrom (8) seitlich in die Innenstruktur (3) eindringen oder aus ihr austreten kann.

3. Medikamentenmagazin nach Anspruch 2, wobei zwei seitliche Öffnungen oder Aussparungen vorgesehen und im wesentlichen senkrecht zueinander angeordnet sind.

4. Medikamentenmagazin nach einem der Ansprüche 2 oder 3, wobei zwei seitliche Öffnungen vorgesehen und auf einer durch die Innenstruktur (3) führenden Achse liegen.

5. Medikamentenmagazin nach einem der Ansprüche 1 - 4, wobei die Innenstruktur (3) ein einstückiges Spritzgusselement ist.

6. Medikamentenmagazin nach einem der vorhergehenden Ansprüche, mit 30 oder 60 Medikamentenkammern, welche 30 bzw. 60 Medikamenten-Einzeldosen entsprechen.

7. Mehrdosispulverinhalator mit einem Medikamentenmagazin nach einem der Ansprüche 1-6.

8. Mehrdosispulverinhalator nach Anspruch 7 zur Applikation eines Arzneimittels, das einen Wirkstoff oder eine Kombination der Wirkstoffe enthält ausgewählt aus der Gruppe Betamimetika, Anticholinergika, Steroide, Antiallergika, Derivate von Mutterkornalkaloiden, Triptane, CGRP-Antagonisten, der Phosphodiesterase -V-Inhibitoren, Phosphodiesterase-IV-Inhibitoren, LTD4-Antagonisten, EGFR-Kinase-Hemmer.

## Claims

1. Medicament magazine having at least one medicament chamber for use in an inhaler, wherein the magazine is formed from two foil strips (1; 2) applied to one another, and the at least one medicament chamber is formed between the foil strips (1; 2), wherein the medicament chamber has an internal structure (3), this internal structure comprising on its inside a volume for accommodating a powdered medicament and for stabilising this inner volume against mechanical influences from the outside, and wherein the internal structure (3) comprises, on a front side, means for opening (4; 11) one of the two foil strips (1; 2),
wherein the means for opening has at least one protruding piercing point or a cutting edge (4,4',4",4"') and protruding opening aids that are set back relative to the piercing point or the cutting edge, and wherein the a retaining device (7) is provided in the medicament magazine which holds the internal structure (3) at the medicament magazine after an opening of the medicament chamber **characterised in that** two parallel cross-members (50) extend, at an inclined angle, from a side wall comprising the cutting edge (4) or the piercing point to a side of the internal structure (3) opposite said side wall.

2. Medicament magazine according to claim 1, wherein the internal structure (3) comprises at least one lateral opening or recess such that an air current (8) can penetrate laterally into an internal structure (3) or can escape therefrom.

3. Medicament magazine according to claim 2, wherein two lateral openings or recesses are provided and arranged essentially perpendicularly to one another.

4. Medicament magazine according to any one of the claims 2 or 3, wherein two lateral openings are provided and located on an axis passing through the internal structure.

5. Medicament magazine according to any one of the claims 1-4, wherein the internal structure (3) is a one-piece injection moulded element.

6. Medicament magazine according to any one of the preceding claims, having 30 or 60 medicament chambers, corresponding to 30 or 60 single doses of medicament.

7. Multi-dose powder inhaler having a medicament magazine according to any one of claims 1-6.

8. Multi-dose powder inhaler according to claim 7, for administering a medicament which contains an active substance or a combination of active substances selected from among: betamimetics, anticholinergics, steroids, antiallergics, ergot alkaloid derivatives, triptanes, CGRP antagonists, phosphodiesterase-V inhibitors, phosphodiesterase-IV inhibitors, LTD4-antagonists, EGFR-kinase inhibitors.

## Revendications

1. Magasin à médicament présentant au moins une chambre à médicament et destiné à être utilisé dans un ensemble d'inhalation,
le magasin étant formé de deux nappes de film (1; 2) appliquées l'une sur l'autre,
la ou les chambres à médicament étant formées entre les nappes de film (1; 2),
la chambre à médicament présentant une structure intérieure (3), laquelle structure intérieure (3) présentant à l'intérieur un volume de reprise d'un médicament (6) en poudre et protégeant ce volume intérieur des effets mécaniques exercés depuis l'extérieur,
la structure intérieure (3) présentant sur un côté avant un moyen (4; 11) d'ouverture d'une des deux nappes de film (1; 2),
le moyen d'ouverture comprenant au moins un point de perforation en saillie ou un tranchant de coupe (4, 4', 4", 4"') et, en retrait par rapport au point de perforation ou au tranchant de coupe, des accessoires d'ouverture débordant,
un moyen de retenue (7) qui retient la structure intérieure (3) sur le magasin à médicament après l'ouverture de la chambre à médicament étant prévu dans le magasin à médicament,
**caractérisé en ce que**
partant d'une paroi qui présente le tranchant de coupe (4) ou le point de perforation latérale, deux nervures transversales parallèles (50) s'étendent sous un angle d'inclinaison en direction d'un côté de la structure intérieure (3) opposé à ladite paroi latérale.

2. Magasin à médicament selon la revendication 1, dans lequel la structure intérieure (3) présente au moins une ouverture ou découpe latérale de telle sorte qu'un écoulement d'air (8) puisse pénétrer dans la structure intérieure (3) ou en sortir.

3. Magasin à médicament selon la revendication 2, dans lequel deux ouvertures ou découpes latérales sont prévues et sont disposées essentiellement perpendiculairement l'une à l'autre.

4. Magasin à médicament selon l'une des revendications 2-3, dans lequel deux ouvertures ou découpes latérales sont prévues et sont disposées sur une axe qui traverse la structure intérieure (3).

5. Magasin à médicament selon l'une des revendications 1 à 4, dans lequel la structure intérieure (3) est un élément d'un seule tenant moulé par injection.

6. Magasin à médicament selon l'une des revendications précédentes, présentant 30 ou 60 chambres à médicament qui correspondent à 30 ou 60 doses unitaires de médicament.

7. Inhalateur de poudre multidoses présentant un magasin à médicament selon l'une des revendications 1 à 6.

8. Inhalateur de poudre multidoses selon la revendication 7, pour l'application d'un médicament qui contient une substance active ou une combinaison de substances actives sélectionnées dans l'ensemble des bêtamimétiques, des anticholinergiques, des stéroïdes, des antiallergiques, des dérivés d'alcaloïdes de l'ergot, des triptanes, des antagonistes du PRGC, des inhibiteurs de la phosphodiestérase-V, des inhibiteurs de la phosphodiestérase-IV, des antagonistes du leucotriène D4 des inhibiteurs de la RFGE-kinase.
